# EUROPEAN PATENT SPECIFICATION

(11) **EP 4 003 255 B1**
(45) Date of publication and mention of the grant of the patent: **24.05.2023**
(21) Application number: 20751298.9
(22) Date of filing: 29.07.2020
(51) Int. Cl.: A61F 13/00, A61F 13/02, A61F 13/06, A61M 1/00

(54) **NEGATIVE-PRESSURE DRESSING FOR FOOT TREATMENT**
UNTERDRUCKVERBAND ZUR FUSSBEHANDLUNG
PANSEMENT À PRESSION NÉGATIVE POUR LE TRAITEMENT D'UN PIED

(30) Priority: 30.07.2019 US 201962880217 P
(43) Date of publication of application: 01.06.2022
(73) Proprietor: KCI Licensing, Inc., San Antonio, TX 78265 (US)
(72) Inventor: LOCKE, Christopher Brian, San Antonio, Texas 78265 (US)
(74) Representative: Simmons & Simmons
(86) International application number: PCT/IB2020/057150
(87) International publication number: WO 2021/019460

(56) References cited:
- US-A1- 2010 087 767
- US-A1- 2018 353 336
- US-A1- 2018 353 337
- US-A1- 2018 353 662

## Description

### CROSS-REFERENCE TO RELATED APPLICATIONS

### TECHNICAL FIELD

The invention set forth in the appended claims relates generally to tissue treatment systems and more particularly, but without limitation, to dressings for tissue treatment.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

### BRIEF SUMMARY

New and useful systems for treating tissue in a negative-pressure therapy environment are set forth in the appended claims. Illustrative embodiments are also provided to enable a person skilled in the art to make and use the claimed subject matter.

The embodiments relate to a dressing comprising: (1) a tissue interface comprising (a) a hindfoot section comprising at least two heel flaps, (b) a midfoot section, (c) a forefoot section, (d) a forefoot extension configured to fold over the forefoot section, (e) a fluid control layer having a plurality of fluid restrictions, and (f) a manifold adhered to the fluid control layer in a stacked relationship across the hindfoot section, the midfoot section, the forefoot section, and the forefoot extension; and (2) a cover comprising a non-porous film having an open end configured to receive the tissue interface applied to the foot. Some embodiments may further comprise a gel layer disposed adjacent to the fluid control layer opposite the manifold, with the gel layer having a plurality of apertures at least partially aligned with the fluid restrictions of the fluid control layer.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide tissue treatment in accordance with this specification;
Figure 2 is an assembly view of an example of a dressing, illustrating additional details that may be associated with some example embodiments of the therapy system of Figure 1;
Figure 3 is a schematic view of an example configuration of fluid restrictions in a layer that may be associated with some embodiments of the dressing of Figure 2;
Figure 4 is a side view of an example of the dressing of Figure 2 that may be associated with some embodiments of the therapy system of Figure 1;
Figure 5 is an assembly view of an example of a dressing, illustrating additional details that may be associated with some example embodiments of the therapy system of Figure 1;
Figure 6 is a schematic view of an example configuration of apertures in a layer that may be associated with some embodiments of the dressing of Figure 5;
Figure 7 is a schematic view of the example layer of Figure 6 overlaid on the example layer of Figure 3;
Figure 8 is an assembly view of an example of a dressing, illustrating additional details that may be associated with some example embodiments of the therapy system of Figure 1;
Figure 9 is an assembly view of an example of a dressing, illustrating additional details that may be associated with some example embodiments of the therapy system of Figure 1;
Figure 10 is an isometric view of an example of an attachment device that may be associated with some example embodiments of the dressing of Figures 2, 5, 8, and 9;
Figure 11 is a plan view of an example of a tissue interface configured for use on a foot, for example to aid in treatment of diabetic foot ulcers or similar wounds;
Figure 12 is a plan view of an alternate example of a tissue interface similar to that of Figure 11, showing exemplary perforation lines that may assist in sizing to a specific user's foot;
Figure 13 is an isometric view of an exemplary cover device that may be associated with some example embodiments of a foot dressing;
Figure 14 is an isometric view of another exemplary cover device that may be associated with some example embodiments of a foot dressing;
Figure 15 is an isometric view of an exemplary cover device with an exemplary separate attachment device that may be associated with some example embodiments of a foot dressing;
Figure 16 is a schematic view of an exemplary tissue interface (for example, as shown in Figures 11-12) with an outline of the bottom of a foot superimposed to demonstrate positioning for application to the foot;
Figure 17 is an isometric view of the tissue interface of Figure 16 when applied to the foot; and
Figure 18 is an isometric view of an exemplary dressing applied to the user's foot, with a cover disposed over the tissue interface positioned on the user's foot.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

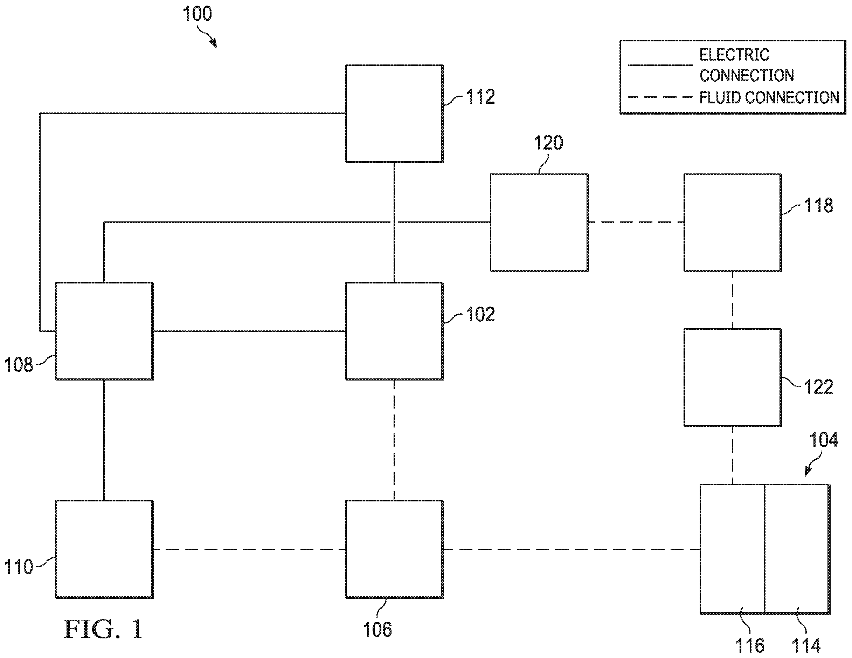

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, a surface wound, bone tissue, adipose tissue, muscle tissue, neural tissue, dermal tissue, vascular tissue, connective tissue, cartilage, tendons, or ligaments. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted. A surface wound, as used herein, is a wound on the surface of a body that is exposed to the outer surface of the body, such as an injury or damage to the epidermis, dermis, and/or subcutaneous layers. Surface wounds may include ulcers or closed incisions, for example. A surface wound, as used herein, does not include wounds within an intra-abdominal cavity. A wound may include chronic, acute, traumatic, subacute, and dehisced wounds, partial-thickness burns, ulcers (such as diabetic, pressure, or venous insufficiency ulcers), flaps, and grafts, for example.

The therapy system 100 may include a source or supply of negative pressure, such as a negative-pressure source 102, a dressing 104, a fluid container, such as a container 106, and a regulator or controller, such as a controller 108, for example. Additionally, the therapy system 100 may include sensors to measure operating parameters and provide feedback signals to the controller 108 indicative of the operating parameters. As illustrated in Figure 1, for example, the therapy system 100 may include a first sensor 110 and a second sensor 112 coupled to the controller 108. As illustrated in the example of Figure 1, the dressing 104 may comprise or consist essentially of one or more dressing layers, such as a tissue interface 114, a cover 116, or both in some embodiments.

The therapy system 100 may also include a source of instillation solution, such as saline, for example. For example, a solution source 118 may be fluidly coupled to the dressing 104, as illustrated in the example embodiment of Figure 1. The solution source 118 may be fluidly coupled to a positive-pressure source such as the positive-pressure source 120, a negative-pressure source such as the negative-pressure source 102, or both in some embodiments. A regulator, such as an instillation regulator 122, may also be fluidly coupled to the solution source 118 and the dressing 104 to ensure proper dosage of instillation solution to a tissue site. For example, the instillation regulator 122 may comprise a piston that can be pneumatically actuated by the negative-pressure source 102 to draw instillation solution from the solution source during a negative-pressure interval and to instill the solution to a dressing during a venting interval. Additionally or alternatively, the controller 108 may be coupled to the negative-pressure source 102, the positive-pressure source 120, or both, to control dosage of instillation solution to a tissue site. In some embodiments, the instillation regulator 122 may also be fluidly coupled to the negative-pressure source 102 through the dressing 104, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the solution source 118, the controller 108 and other components into a therapy unit.

In general, components of the therapy system 100 may be coupled directly or indirectly. For example, the negative-pressure source 102 may be directly coupled to the container 106, and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 108. The negative-pressure source maybe fluidly coupled to one or more distribution components, which provide a fluid path to a tissue site. In some embodiments, components may also be coupled by virtue of physical proximity, being integral to a single structure, or being formed from the same piece of material. For example, the tissue interface 114 and the cover 116 may be discrete layers disposed adjacent to each other, and may be joined together in some embodiments.

A distribution component is preferably detachable, and may be disposable, reusable, or recyclable. The dressing 104 and the container 106 are illustrative of distribution components. A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components, including sensors and data communication devices. In some embodiments, for example, a dressing interface may facilitate coupling a fluid conductor to the dressing 104. For example, such a dressing interface may be a SENSAT.R.A.C.^{™} Pad available from KCI of San Antonio, Texas.

A negative-pressure supply, such as the negative-pressure source 102, may be a reservoir of air at a negative pressure, or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-9.9 kPa) and -300 mm Hg (-39.9 kPa).

The container 106 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container can reduce waste and costs associated with negative-pressure therapy.

A controller, such as the controller 108, may be a microprocessor or computer programmed to operate one or more components of the therapy system 100, such as the negative-pressure source 102. In some embodiments, for example, the controller 108 may be a microcontroller, which generally comprises an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the tissue interface 114, for example. The controller 108 is also preferably configured to receive one or more input signals, such as a feedback signal, and programmed to modify one or more operating parameters based on the input signals.

Sensors, such as the first sensor 110 and the second sensor 112, are generally known in the art as any apparatus operable to detect or measure a physical phenomenon or property, and generally provide a signal indicative of the phenomenon or property that is detected or measured. For example, the first sensor 110 and the second sensor 112 may be configured to measure one or more operating parameters of the therapy system 100. In some embodiments, the first sensor 110 may be a transducer configured to measure pressure in a pneumatic pathway and convert the measurement to a signal indicative of the pressure measured. In some embodiments, for example, the first sensor 110 may be a piezo-resistive strain gauge. The second sensor 112 may optionally measure operating parameters of the negative-pressure source 102, such as the voltage or current, in some embodiments. Preferably, the signals from the first sensor 110 and the second sensor 112 are suitable as an input signal to the controller 108, but some signal conditioning may be appropriate in some embodiments. For example, the signal may need to be filtered or amplified before it can be processed by the controller 108. Typically, the signal is an electrical signal, but may be represented in other forms, such as an optical signal.

The tissue interface 114 can be generally adapted to contact a tissue site. The tissue interface 114 may be partially or fully in contact with the tissue site. If the tissue site is a wound, for example, the tissue interface 114 may partially or completely fill the wound, or may be placed over the wound. The tissue interface 114 may take many forms and have more than one layer in some embodiments. The tissue interface 114 may also have many sizes, shapes, or thicknesses depending on a variety of factors, such as the type of treatment being implemented or the nature and size of a tissue site. For example, the size and shape of the tissue interface 114 may be adapted to the contours of deep and irregular shaped tissue sites.

In some embodiments, the cover 116 may provide a bacterial barrier and protection from physical trauma. The cover 116 may also be constructed from a material that can reduce evaporative losses and provide a fluid seal between two components or two environments, such as between a therapeutic environment and a local external environment. The cover 116 may comprise or consist of, for example, an elastomeric film or membrane that can provide a seal adequate to maintain a negative pressure at a tissue site for a given negative-pressure source. The cover 116 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide may provide effective breathability and mechanical properties.

In some example embodiments, the cover 116 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. For example, the cover 116 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Coveris Advanced Coatings, Wrexham, United Kingdom. In some embodiments, the cover 116 may comprise INSPIRE 2301 having an MVTR (upright cup technique) of 2600 g/m2/24 hours and a thickness of about 30 microns.

An attachment device may be used to attach the cover 116 to an attachment surface, such as undamaged epidermis, a gasket, or another cover. The attachment device may take many forms. For example, an attachment device may be a medically-acceptable, pressure-sensitive adhesive configured to bond the cover 116 to epidermis around a tissue site, such as a surface wound. In some embodiments, for example, the adhesive may be an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 118 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

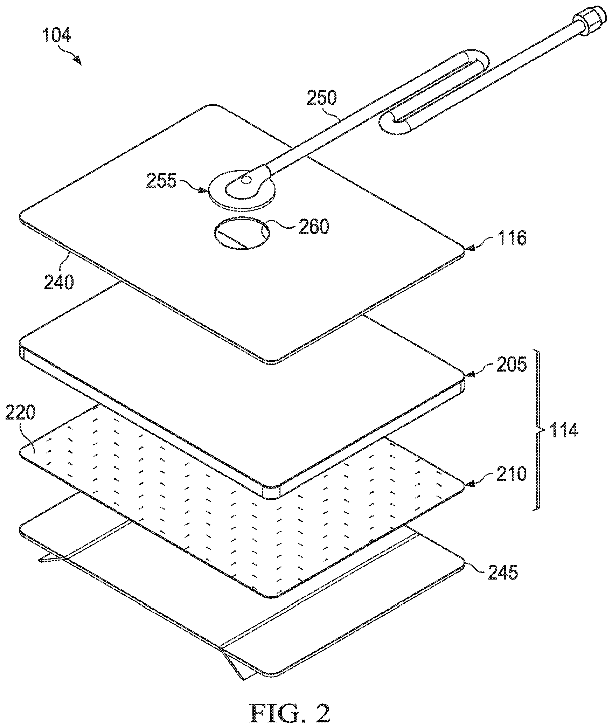

Figure 2 is an assembly view of an example of the dressing 104 of Figure 1, illustrating additional details that may be associated with some embodiments in which the tissue interface 114 comprises more than one layer. In the example of Figure 2, the tissue interface comprises a first layer 205 and a second layer 210. In some embodiments, the first layer 205 may be disposed adjacent to the second layer 210. For example, the first layer 205 and the second layer 210 may be stacked so that the first layer 205 is in contact with the second layer 210. The first layer 205 may also be heat-bonded or adhered to the second layer 210 in some embodiments.

The first layer 205 generally comprises or consists essentially of a manifold or a manifold layer, which provides a means for collecting or distributing fluid across the tissue interface 114 under pressure. For example, the first layer 205 may be adapted to receive negative pressure from a source and distribute negative pressure through multiple apertures across the tissue interface 114, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 114.

In some illustrative embodiments, the pathways of the first layer 205 may be interconnected to improve distribution or collection of fluids. In some illustrative embodiments, the first layer 205 may comprise or consist essentially of a porous material having interconnected fluid pathways. For example, open-cell foam, porous tissue collections, and other porous material such as gauze or felted mat generally include pores, edges, and/or walls adapted to form interconnected fluid channels. Other suitable materials may include a 3D textile (Baltex, Muller, Heathcoates), non-woven (Libeltex, Freudenberg), a 3D polymeric structure (molded polymers, embossed and formed films, and fusion bonded films [Supracore]), and mesh, for example. Liquids, gels, and other foams may also include or be cured to include apertures and fluid pathways. In some embodiments, the first layer 205 may additionally or alternatively comprise projections that form interconnected fluid pathways. For example, the first layer 205 may be molded to provide surface projections that define interconnected fluid pathways. Any or all of the surfaces of the first layer 205 may have an uneven, coarse, or jagged profile

In some embodiments, the first layer 205 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns may be particularly suitable for some types of therapy. The tensile strength of the first layer 205 may also vary according to needs of a prescribed therapy. For example, the tensile strength of the first layer 205 may be increased for instillation of topical treatment solutions. The 25% compression load deflection of the first layer 205 may be at least 0.35 pounds per square inch, 2413 Pa (0.35 pounds per square inch), and the 65% compression load deflection may be at least 2965 Pa (0.43 pounds per square inch). In some embodiments, the tensile strength of the first layer 205 may be at least 10 pounds per square inch. The first layer 205 may have a tear strength of at least 17237 Pa (2.5 pounds per square inch). In some embodiments, the first layer 205 may be foam comprised of polyols such as polyester or polyether, isocyanate such as toluene diisocyanate, and polymerization modifiers such as amines and tin compounds. In one non-limiting example, the first layer 205 may be a reticulated polyurethane foam such as used in GRANUFOAM^{™} dressing or V.A.C. VERAFLO^{™} dressing, both available from KCI of San Antonio, Texas.

The first layer 205 generally has a first planar surface and a second planar surface opposite the first planar surface. The thickness of the first layer 205 between the first planar surface and the second planar surface may also vary according to needs of a prescribed therapy. For example, the thickness of the first layer 205 may be decreased to relieve stress on other layers and to reduce tension on peripheral tissue. The thickness of the first layer 205 can also affect the conformability of the first layer 205. In some embodiments, a thickness in a range of about 5 millimeters to 10 millimeters may be suitable.

The second layer 210 may comprise or consist essentially of a means for controlling or managing fluid flow, for example a fluid control layer. In some embodiments, the second layer 210 may comprise or consist essentially of a liquid-impermeable, elastomeric material. For example, the second layer 210 may comprise or consist essentially of a polymer film. The second layer 210 may also have a smooth or matte surface texture in some embodiments. A glossy or shiny finish better or equal to a grade B3 according to the SPI (Society of the Plastics Industry) standards may be particularly advantageous for some applications. In some embodiments, variations in surface height may be limited to acceptable tolerances. For example, the surface of the second layer may have a substantially flat surface, with height variations limited to 0.2 millimeters over a centimeter.

In some embodiments, the second layer 210 may be hydrophobic. For example, in some embodiments, the contact angle of the second layer 210 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Non-limiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles reported herein represent averages of 5-9 measured values, discarding both the highest and lowest measured values. The hydrophobicity of the second layer 210 may be further enhanced with a hydrophobic coating of other materials, such as silicones and fluorocarbons, either as coated from a liquid, or plasma coated.

In some embodiments, the second layer 210 may be formed of a hydrophilic polymer film. The hydrophilic polymer film may have a water contact angle of at least about 65 degrees to about 90 degrees. The hydrophilic polymer film may include polyurethane. In some embodiments, the second layer 210 may have a first surface that faces a tissue site when the dressing 104 faces a tissue site, and a second surface opposite the first surface. The first surface may be a hydrophilic surface and the second surface may be a hydrophobic surface. The hydrophobic surface may discourage fluid from moving back towards a tissue site once the fluid has moved into the first layer 205. The second layer 210 having a hydrophilic surface and a hydrophobic surface may be obtained through lamination of a hydrophobic film to a hydrophilic film, or through plasma/corona surface treatments of a single film type.

The second layer 210 may also be suitable for welding to other layers, including the first layer 205. For example, the second layer 210 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene.

The area density of the second layer 210 may vary according to a prescribed therapy or application. In some embodiments, an area density of less than 40 grams per square meter may be suitable, and an area density of about 20-30 grams per square meter may be particularly advantageous for some applications.

In some embodiments, for example, the second layer 210 may comprise or consist essentially of a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. Other suitable polymeric films include polyurethanes, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styreneics, silicones, fluoropolymers, and acetates. A thickness between 20 microns and 100 microns may be suitable for many applications. Films may be clear, colored, or printed. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

As illustrated in the example of Figure 2, the second layer 210 may have one or more fluid restrictions 220, which can be distributed uniformly or randomly across the second layer 210. The fluid restrictions 220 may be bi-directional and pressure-responsive. For example, each of the fluid restrictions 220 generally may comprise or consist essentially of an elastic passage that is normally unstrained to substantially reduce liquid flow, and can expand or open in response to a pressure gradient. In some embodiments, the fluid restrictions 220 may comprise or consist essentially of perforations in the second layer 210. Perforations may be formed by removing material from the second layer 210. For example, perforations may be formed by cutting through the second layer 210, which may also deform the edges of the perforations in some embodiments. In the absence of a pressure gradient across the perforations, the passages may be sufficiently small to form a seal or fluid restriction, which can substantially reduce or prevent liquid flow. Additionally or alternatively, one or more of the fluid restrictions 220 may be an elastomeric valve that is normally closed when unstrained to substantially prevent liquid flow, and can open in response to a pressure gradient. A fenestration in the second layer 210 may be a suitable valve for some applications. Fenestrations may also be formed by removing material from the second layer 210, but the amount of material removed and the resulting dimensions of the fenestrations may be up to an order of magnitude less than perforations, and may not deform the edges.

For example, some embodiments of the fluid restrictions 220 may comprise or consist essentially of one or more slits, slots or combinations of slits and slots in the second layer 210. In some examples, the fluid restrictions 220 may comprise or consist of linear slots having a length less than 4 millimeters and a width less than 1 millimeter. The length may be at least 2 millimeters, and the width may be at least 0.4 millimeters in some embodiments. A length of about 3 millimeters and a width of about 0.8 millimeters may be particularly suitable for many applications, and a tolerance of about 0.1 millimeter may also be acceptable. Such dimensions and tolerances may be achieved with a laser cutter, for example. Slots of such configurations may function as imperfect valves that substantially reduce liquid flow in a normally closed or resting state. For example, such slots may form a flow restriction without being completely closed or sealed. The slots can expand or open wider in response to a pressure gradient to allow increased liquid flow.

In the example of Figure 2, the dressing 104 may further include an attachment device, such as an adhesive 240. The adhesive 240 may be, for example, a medically-acceptable, pressure-sensitive adhesive that extends about a periphery, a portion, or the entire cover 116. In some embodiments, for example, the adhesive 240 may be an acrylic adhesive having a coating weight between 25-65 grams per square meter (g.s.m.). Thicker adhesives, or combinations of adhesives, may be applied in some embodiments to improve the seal and reduce leaks. In some embodiments, such a layer of the adhesive 240 may be continuous or discontinuous. Discontinuities in the adhesive 240 may be provided by apertures or holes (not shown) in the adhesive 240. The apertures or holes in the adhesive 240 may be formed after application of the adhesive 240 or by coating the adhesive 240 in patterns on a carrier layer, such as, for example, a side of the cover 116. Apertures or holes in the adhesive 240 may also be sized to enhance the MVTR of the dressing 104 in some example embodiments.

As illustrated in the example of Figure 2, in some embodiments, the dressing 104 may include a release liner 245 to protect the adhesive 240 prior to use. The release liner 245 may also provide stiffness to assist with, for example, deployment of the dressing 104. The release liner 245 may be, for example, a casting paper, a film, or polyethylene. Further, in some embodiments, the release liner 245 may be a polyester material such as polyethylene terephthalate (PET), or similar polar semi-crystalline polymer. The use of a polar semi-crystalline polymer for the release liner 245 may substantially preclude wrinkling or other deformation of the dressing 104. For example, the polar semi-crystalline polymer may be highly orientated and resistant to softening, swelling, or other deformation that may occur when brought into contact with components of the dressing 104, or when subjected to temperature or environmental variations, or sterilization. Further, a release agent may be disposed on a side of the release liner 245 that is configured to contact the second layer 210. For example, the release agent may be a silicone coating and may have a release factor suitable to facilitate removal of the release liner 245 by hand and without damaging or deforming the dressing 104. In some embodiments, the release agent may be a fluorocarbon or a fluorosilicone, for example. In other embodiments, the release liner 245 may be uncoated or otherwise used without a release agent.

Figure 2 also illustrates one example of a fluid conductor 250 and a dressing interface 255. As shown in the example of Figure 2, the fluid conductor 250 may be a flexible tube, which can be fluidly coupled on one end to the dressing interface 255. The dressing interface 255 may be an elbow connector, as shown in the example of Figure 2, which can be placed over an aperture 260 in the cover 116 to provide a fluid path between the fluid conductor 250 and the tissue interface 114.

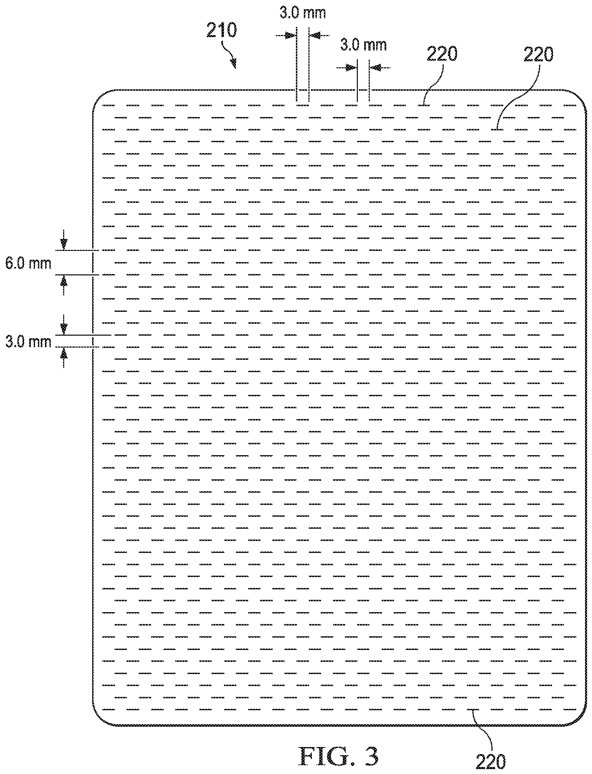

Figure 3 is a schematic view of an example of the second layer 210, illustrating additional details that may be associated with some embodiments. As illustrated in the example of Figure 3, the fluid restrictions 220 may each consist essentially of one or more linear slots having a length of about 3 millimeters. Figure 3 additionally illustrates an example of a uniform distribution pattern of the fluid restrictions 220. In Figure 3, the fluid restrictions 220 are substantially coextensive with the second layer 210, and are distributed across the second layer 210 in a grid of parallel rows and columns, in which the slots are also mutually parallel to each other. In some embodiments, the rows may be spaced about 3 millimeters on center, and the fluid restrictions 220 within each of the rows may be spaced about 3 millimeters on center as illustrated in the example of Figure 3. The fluid restrictions 220 in adjacent rows may be aligned or offset. For example, adjacent rows may be offset, as illustrated in Figure 3, so that the fluid restrictions 220 are aligned in alternating rows and separated by about 6 millimeters. The spacing of the fluid restrictions 220 may vary in some embodiments to increase the density of the fluid restrictions 220 according to therapeutic requirements.

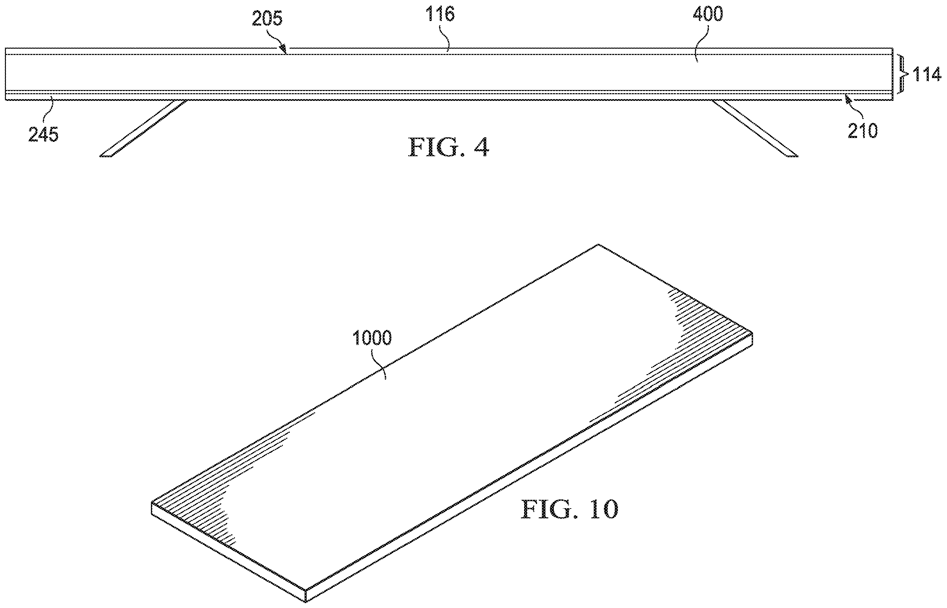

Figure 4 is a side view of an example of the dressing of Figure 2 that may be associated with some embodiments of the therapy system of Figure 1. As shown in Figure 4, the dressing 104 has an exposed perimeter 400 (exposed edges). More particularly, in the example of Figure 4 the first layer 205, the cover 116, and the second layer 210 each have an exposed perimeter, and there is no seam, weld, or seal along the exposed perimeter 400.

One or more of the components of the dressing 104 may additionally be treated with an antimicrobial agent in some embodiments. For example, the first layer 205 may be a foam, mesh, or non-woven coated with an antimicrobial agent. In some embodiments, the first layer may comprise antimicrobial elements, such as fibers coated with an antimicrobial agent. Additionally or alternatively, some embodiments of the second layer 210 may be a polymer coated or mixed with an antimicrobial agent. In other examples, the fluid conductor 250 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Additionally or alternatively, one or more of the components may be coated with a mixture that may include citric acid and collagen, which can reduce bio-films and infections. For example, the first layer 205 may be foam coated with such a mixture.

Individual components of the dressing 104 may be bonded or otherwise secured to one another with a solvent or non-solvent adhesive, or with thermal welding, for example, without adversely affecting fluid management. For example, the cover 116 may be laminated to the first layer 205, and the second layer 210 may be laminated to the first layer 205 opposite the cover 116 in some embodiments. In some embodiments, the second layer 210 may be a polyurethane film that is heat-bonded to the first layer 205, which may be polyurethane foam.

The cover 116, the first layer 205, and the second layer 210, or various combinations may be assembled before application or in situ. The second layer 210 may provide a smooth surface opposite the first layer 205. In some embodiments, one or more layers of the tissue interface 114 may coextensive. For example, the second layer 210 may be cut flush with the edge of the first layer 205, exposing the edge of the first layer 205, as illustrated in the embodiment of Figure 2. In other embodiments, the second layer 210 may overlap the edge of the first layer 205. In some embodiments, the dressing 104 may be provided as a single, composite dressing. For example, the second layer 210 may be coupled to the cover 116 to enclose the first layer 205, wherein the second layer 210 is configured to face a tissue site.

In use, the release liner 245 (if included) may be removed to expose the second layer 210, which may be placed within, over, on, or otherwise proximate to a tissue site, particularly a surface tissue site and adjacent epidermis. The second layer 210 may be interposed between the first layer 205 and the tissue site and adjacent epidermis, which can substantially reduce or eliminate adverse interaction with the first layer 205. For example, the second layer 210 may be placed over a surface wound (including edges of the wound) and undamaged epidermis to prevent direct contact with the first layer 205. Treatment of a surface wound or placement of the dressing 104 on a surface wound includes placing the dressing 104 immediately adjacent to the surface of the body or extending over at least a portion of the surface of the body. Treatment of a surface wound does not include placing the dressing 104 wholly within the body or wholly under the surface of the body, such as placing a dressing within an abdominal cavity. The cover 116 may be sealed to an attachment surface, such as epidermis peripheral to a tissue site, to provide an effective seal around the first layer 205 and the second layer 210. For example, a suitable seal for some applications may limit flow to less than about 950 cc/minute.

The geometry and dimensions of the tissue interface 114, the cover 116, or both may vary to suit a particular application or anatomy. For example, the geometry or dimensions of the tissue interface 114 and the cover 116 may be adapted to provide an effective and reliable seal against challenging anatomical surfaces, such as an elbow or heel, at and around a tissue site. Additionally or alternatively, the dimensions may be modified to increase the surface area for the second layer 210 to enhance the movement and proliferation of epithelial cells at a tissue site and reduce the likelihood of granulation tissue in-growth.

Thus, the dressing 104 in the example of Figure 2 can provide a sealed therapeutic environment proximate to a tissue site, substantially isolated from the external environment, and the negative-pressure source 102 can reduce the pressure in the sealed therapeutic environment. Negative pressure in the sealed environment may compress the first layer 205 into the second layer 210, which can deform the surface of the second layer 210 to provide an uneven, coarse, or jagged profile that can induce macrostrain and micro-strain in the tissue site in some embodiments. Negative pressure applied through the tissue interface 114 can also create a negative pressure differential across the fluid restrictions 220 in the second layer 210, which can open the fluid restrictions 220 to allow exudate and other liquid movement through the fluid restrictions 220 into the first layer 205 and the container 106. For example, in some embodiments in which the fluid restrictions 220 may comprise perforations through the second layer 210, a pressure gradient across the perforations can strain the adjacent material of the second layer 210 and increase the dimensions of the perforations to allow liquid movement through them, similar to the operation of a duckbill valve.

In some embodiments, the first layer 205 may be hydrophobic to minimize retention or storage of liquid in the dressing 104. In other embodiments, the first layer 205 may be hydrophilic. In an example in which the first layer 205 may be hydrophilic, the first layer 205 may also wick fluid away from a tissue site, while continuing to distribute negative pressure to the tissue site. The wicking properties of the first layer 205 may draw fluid away from a tissue site by capillary flow or other wicking mechanisms, for example. Polyvinyl alcohol, open-cell foam such as V.A.C. WHITEFOAM^{™} dressing available from KCI of San Antonio, Texas is an example of a hydrophilic foam that may be suitable for some examples of the first layer 205. Other hydrophilic foams may include those made from polyether. Other foams that may exhibit hydrophilic characteristics include hydrophobic foams that have been treated or coated to provide hydrophilicity.

If the negative-pressure source 102 is removed or turned-off, the pressure differential across the fluid restrictions 220 can dissipate, allowing the fluid restrictions 220 to return to an unstrained or resting state and prevent or reduce the return rate of exudate or other liquid moving to the tissue site through the second layer 210.

In some applications, a filler may also be disposed between a tissue site and the second layer 210. For example, if the tissue site is a surface wound, a wound filler may be applied interior to the periwound, and the second layer 210 may be disposed over the periwound and the wound filler. In some embodiments, the filler may be a manifold, such as an open-cell foam. The filler may comprise or consist essentially of the same material as the first layer 205 in some embodiments.

Additionally or alternatively, the tissue interface 114 may be formed into strips suitable for use as bridges or to fill tunnel wounds, for example. Strips having a width of about 5 millimeters to 30 millimeters may be suitable for some embodiments.

Additionally or alternatively, the second layer 210 may comprise reinforcing fibers to increase its tensile strength, which may be advantageous for use in tunnel wounds.

Additionally or alternatively, instillation solution or other fluid may be distributed to the dressing 104, which can increase the pressure in the tissue interface 114. The increased pressure in the tissue interface 114 can create a positive pressure differential across the fluid restrictions 220 in the second layer 210, which can open or expand the fluid restrictions 220 from their resting state to allow the instillation solution or other fluid to be distributed to the tissue site.

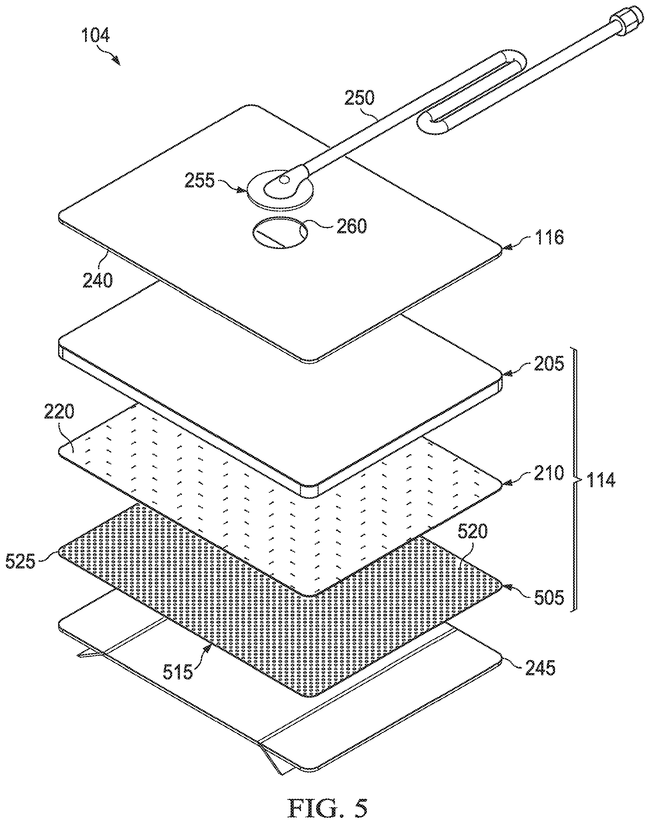

Figure 5 is an assembly view of another example of the dressing 104 of Figure 1, illustrating additional details that may be associated with some embodiments in which the tissue interface 114 may comprise additional layers. In the example of Figure 5, the tissue interface 114 comprises a third layer 505 in addition to the second layer 210. In some embodiments, the third layer 505 may be adjacent to the second layer 210 opposite the first layer 205. The third layer 505 may also be bonded to the second layer 210 in some embodiments.

The third layer 505 may comprise or consist essentially of a sealing layer formed from a soft, pliable material, such as a tacky gel, suitable for providing a fluid seal with a tissue site, and may have a substantially flat surface. Thus, the third layer 505 may comprise or consist essentially of a gel layer in some embodiments. For example, the third layer 505 may comprise, without limitation, a silicone gel, a soft silicone, hydrocolloid, hydrogel, polyurethane gel, polyolefin gel, hydrogenated styrenic copolymer gel, a foamed gel, a soft closed cell foam such as polyurethanes and polyolefins coated with an adhesive, polyurethane, polyolefin, or hydrogenated styrenic copolymers. The third layer 505 may include an adhesive surface on an underside and a patterned coating of acrylic on a top side. The patterned coating of acrylic may be applied about a peripheral area to allow higher bonding in regions that are likely to be in contact with skin rather than the wound area. In other embodiments, the third layer 505 may comprise a low-tack adhesive layer instead of silicone. In some embodiments, the third layer 505 may have a thickness between about 200 microns (µm) and about 1000 microns (µm). In some embodiments, the third layer 505 may have a hardness between about 5 Shore OO and about 80 Shore OO. Further, the third layer 505 may be comprised of hydrophobic or hydrophilic materials.

In some embodiments, the third layer 505 may be a hydrophobic-coated material. For example, the third layer 505 may be formed by coating a spaced material, such as, for example, woven, nonwoven, molded, or extruded mesh with a hydrophobic material. The hydrophobic material for the coating may be a soft silicone, for example.

The third layer 505 may have corners 525 and edges 515. The third layer 505 may include apertures 520. The apertures 520 may be formed by cutting or by application of local RF or ultrasonic energy, for example, or by other suitable techniques for forming an opening. The apertures 520 may have a uniform distribution pattern, or may be randomly distributed on the third layer 505. The apertures 520 in the third layer 505 may have many shapes, including circles, squares, stars, ovals, polygons, slits, complex curves, rectilinear shapes, triangles, for example, or may have some combination of such shapes.

Each of the apertures 520 may have uniform or similar geometric properties. For example, in some embodiments, each of the apertures 520 may be circular apertures, having substantially the same diameter. In some embodiments, the diameter of each of the apertures 520 may be between about 1 millimeter and about 50 millimeters. In other embodiments, the diameter of each of the apertures 520 may be between about 1 millimeter and about 20 millimeters.

In other embodiments, geometric properties of the apertures 520 may vary. For example, the diameter of the apertures 520 may vary depending on the position of the apertures 520 in the third layer 505. The apertures 520 may be spaced substantially equidistant over the third layer 505. Alternatively, the spacing of the apertures 520 may be irregular.

As illustrated in the example of Figure 5, in some embodiments, the release liner 245 may be attached to or positioned adjacent to the third layer 505 to protect the adhesive 240 prior to use. In some embodiments, the release liner 245 may have a surface texture that may be imprinted on an adjacent layer, such as the third layer 505. Further, a release agent may be disposed on a side of the release liner 245 that is configured to contact the third layer 505.

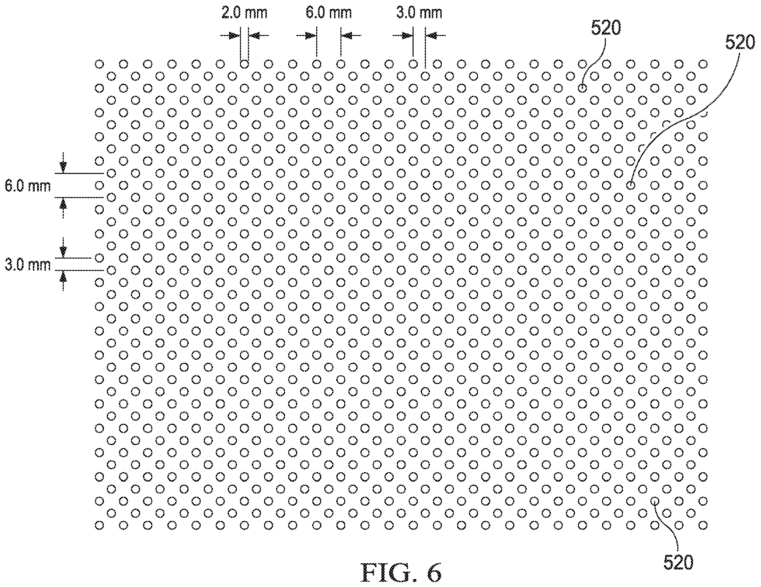

Figure 6 is a schematic view of an example configuration of the apertures 520, illustrating additional details that may be associated with some embodiments of the third layer 505. In some embodiments, the apertures 520 illustrated in Figure 6 may be associated only with an interior portion of the third layer 505. In the example of Figure 6, the apertures 520 are generally circular and have a diameter of about 2 millimeters. Figure 6 also illustrates an example of a uniform distribution pattern of the apertures 520. In Figure 6, the apertures 520 are distributed across the third layer 505 in a grid of parallel rows and columns. Within each row and column, the apertures 520 may be equidistant from each other, as illustrated in the example of Figure 6. Figure 6 illustrates one example configuration that may be particularly suitable for many applications, in which the apertures 520 are spaced about 6 millimeters apart along each row and column, with a 3 millimeter offset.

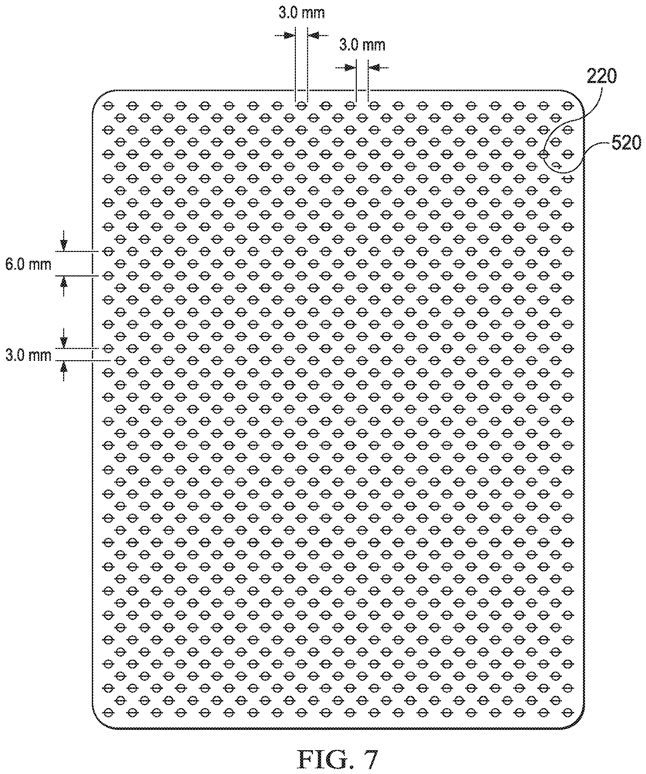

Figure 7 is a schematic view of the third layer 505 of Figure 6 overlaid on the second layer 210 of Figure 3, illustrating additional details that may be associated with some example embodiments of the tissue interface 114. For example, as illustrated in Figure 7, the fluid restrictions 220 may be aligned, overlapping, in registration with, or otherwise fluidly coupled to the apertures 520 in some embodiments. In some embodiments, one or more of the fluid restrictions 220 may be registered with the apertures 520 only in an interior portion, or only partially registered with the apertures 520. The fluid restrictions 220 in the example of Figure 7 are generally configured so that each of the fluid restrictions 220 is registered with only one of the apertures 520. In other examples, one or more of the fluid restrictions 220 may be registered with more than one of the apertures 520. For example, any one or more of the fluid restrictions 220 may be a perforation or a fenestration that extends across two or more of the apertures 520. Additionally or alternatively, one or more of the fluid restrictions 220 may not be registered with any of the apertures 520.

As illustrated in the example of Figure 7, the apertures 520 may be sized to expose a portion of the second layer 210, the fluid restrictions 220, or both through the third layer 505. In some embodiments, one or more of the apertures 520 may be sized to expose more than one of the fluid restrictions 220. For example, some or all of the apertures 520 may be sized to expose two or three of the fluid restrictions 220. In some examples, the length of each of the fluid restrictions 220 may be substantially equal to the diameter of each of the apertures 520. More generally, the average dimensions of the fluid restrictions 220 are substantially similar to the average dimensions of the apertures 520. For example, the apertures 520 may be elliptical in some embodiments, and the length of each of the fluid restrictions 220 may be substantially equal to the major axis or the minor axis. In some embodiments, though, the dimensions of the fluid restrictions 220 may exceed the dimensions of the apertures 520, and the size of the apertures 520 may limit the effective size of the fluid restrictions 220 exposed to the lower surface of the dressing 104.

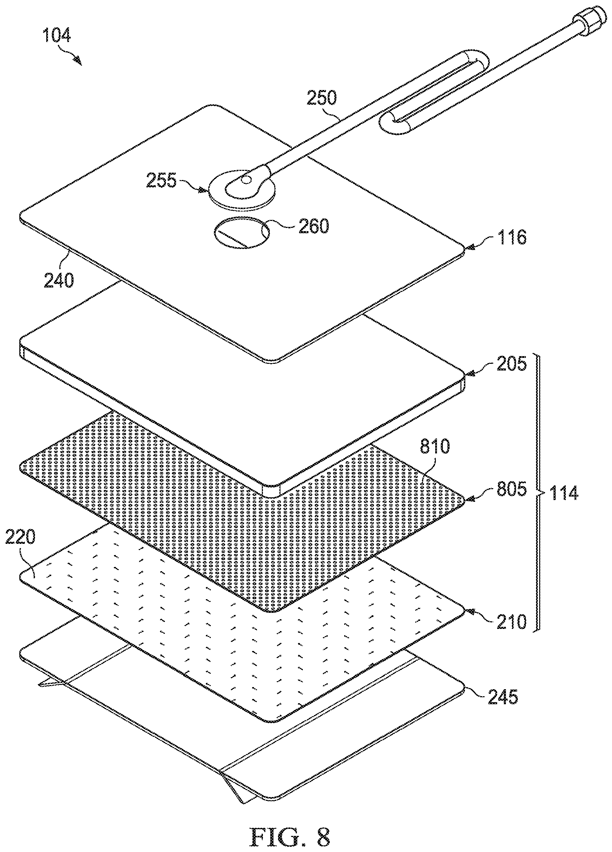

Figure 8 is an assembly view of an example of the dressing 104, illustrating additional details that may be associated with some example embodiments of the therapy system of Figure 1 in which the dressing 104 may comprise a tie layer 805 in addition to the first layer 205 and the second layer 210. The tie layer 805 may have perforations 810 and may have a thickness between 10 microns and 100 microns in some embodiments. The tie layer 805 may be clear, colored, or printed. As illustrated in Figure 8, the tie layer 805 may be disposed between the first layer 205 and the second layer 210. The third layer 505 may also be bonded to at least one of the first layer 205 and the second layer 210 in some embodiments.

The tie layer 805 may comprise polyurethane film, for example, which can be bonded to the first layer 205 and the second layer 210. For example, if the first layer 205 is polyurethane foam and the second layer 210 is formed of a polyethylene film, the second layer 210 may be more readily bonded to the tie layer 805 than directly to the first layer 205.

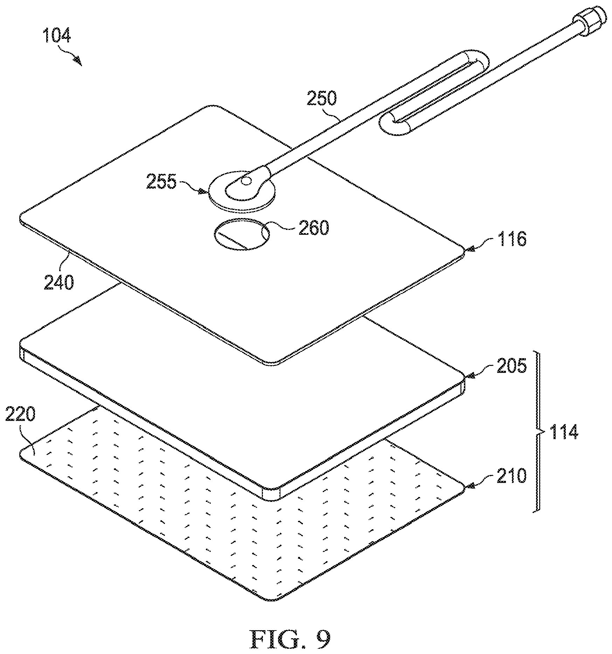

Figure 9 is an assembly view of an example of the dressing 104, illustrating additional details that may be associated with some example embodiments of the therapy system of Figures 1 in which the dressing 104 may comprise the first layer 205, the second layer 210, and the cover 116 only. In some embodiments, the second layer 210 optionally includes a low tack adhesive on the first side. The low tack adhesive may be configured to hold the dressing 104 in place while the cover 116 is applied. The low tack adhesive may be continuously coated on the second layer 210 or applied in a pattern.

Figure 10 is a perspective view of an example of an attachment device 1000 that may be associated with some example embodiments of the dressing 104. In some embodiments, the attachment device 1000 may include one or more polymer strips, such as polyurethane strips, having an adhesive thereon. The attachment device 1000 can be configured to seal the exposed perimeter 400 and affix the dressing 104 to a patient's skin so as to provide both a seal and long-term mechanical fixation. The attachment device 1000 may also be applied to areas when the dressing 104 has been compromised due to the need to form a 3-dimensional shape. In some embodiments, the attachment device 1000 may be a composite strip of a perforated gel, substantially similar to the third layer 505 and a backing with an acrylic adhesive.

The cover 116, the first layer 205, the second layer 210, the third layer 505, or various combinations may be assembled before application or in situ. For example, the cover 116 may be laminated to the first layer 205, and the second layer 210 may be laminated to the first layer 205 opposite the cover 116 in some embodiments. The third layer 505 may also be coupled to the second layer 210 opposite the first layer 205 in some embodiments. In some embodiments, one or more layers of the tissue interface 114 may coextensive. For example, the second layer 210, the third layer 505, the tie layer 805, or any combination thereof may be cut flush with the edge of the first layer 205, exposing the edge of the first layer 205. In other embodiments, the second layer 210, the third layer 505, the tie layer 805, or any combination thereof may overlap the edge of the first layer 205.

In use, the dressing 104 may be sized to a specific region or anatomical area through cutting to manage radii, such as for amputations. The dressing 104 may be cut without losing pieces of the dressing 104 and without the dressing 104 falling apart. Once the dressing 104 is shaped to the anatomical or wound area, the release liner 245 (if included) may be removed to expose the third layer 505 of the example of Figure 5, and the dressing 104 may be placed within, over, on, or otherwise proximate to a tissue site, particularly a surface tissue site and adjacent epidermis. The third layer 505, when formed of silicone, may provide a temporary fixation.

The third layer 505, the tie layer 805, and the second layer 210 may be interposed between the first layer 205 and the tissue site, which can substantially reduce or eliminate adverse interaction with the first layer 205. For example, the third layer 505 may be placed over a surface wound (including edges of the wound) and undamaged epidermis to prevent direct contact with the first layer 205. In some applications, the interior portion of the third layer 505 may be positioned adjacent to, proximate to, or covering a tissue site. In some applications, at least some portion of the second layer 210, the fluid restrictions 220, or both may be exposed to a tissue site through the third layer 505. The periphery of the third layer 505 may be positioned adjacent to or proximate to tissue around or surrounding the tissue site. The third layer 505 may be sufficiently tacky to hold the dressing 104 in position prior to application of the attachment device 1000, while also allowing the dressing 104 to be removed or re-positioned without trauma to the tissue site.

In some embodiments, the tissue interface 114 is applied to a wound before the cover 116 is applied over the tissue interface 114, and a hole is cut in the cover 116. In some embodiments, the second layer 210 having a low tack adhesive on a tissue facing side permits the dressing to be held in place while the cover 116 is applied over the tissue interface 114.

The geometry and dimensions of the tissue interface 114, the cover 116, or both may vary to suit a particular application or anatomy. Additionally or alternatively, the dimensions may be modified to increase the surface area for the third layer 505 to enhance the movement and proliferation of epithelial cells at a tissue site and reduce the likelihood of granulation tissue in-growth.

Further, the dressing 104 and the attachment device 1000 may permit re-application or repositioning to reduce or eliminate leaks, which can be caused by creases and other discontinuities in the dressing 104 or a tissue site. The ability to rectify leaks may increase the reliability of the therapy and reduce power consumption in some embodiments.

Thus, the dressing 104 can provide a sealed therapeutic environment proximate to a tissue site, substantially isolated from the external environment, and the negative-pressure source 102 can reduce the pressure in the sealed therapeutic environment.

If not already configured, the dressing interface 255 may be disposed over the aperture 260 and attached to the cover 116. The fluid conductor 250 may be fluidly coupled to the dressing interface 255 and to the negative-pressure source 102.

Negative pressure applied through the tissue interface 114 can create a negative pressure differential across the fluid restrictions 220 in the second layer 210, which can open or expand the fluid restrictions 220. For example, in some embodiments in which the fluid restrictions 220 may comprise substantially closed fenestrations through the second layer 210, a pressure gradient across the fenestrations can strain the adjacent material of the second layer 210 and increase the dimensions of the fenestrations to allow liquid movement through them, similar to the operation of a duckbill valve. Opening the fluid restrictions 220 can allow exudate and other liquid movement through the fluid restrictions 220 into the first layer 205 and the container 106. Changes in pressure can also cause the first layer 205 to expand and contract, and the interior border 435 may protect the epidermis from irritation. The second layer 210 and the third layer 505 can also substantially reduce or prevent exposure of tissue to the first layer 205, which can inhibit growth of tissue into the first layer 205.

If the negative-pressure source 102 is removed or turned off, the pressure differential across the fluid restrictions 220 can dissipate, allowing the fluid restrictions 220 to close and prevent exudate or other liquid from returning to the tissue site through the second layer 210.

In some applications, a filler may also be disposed between a tissue site and the third layer 505. For example, if the tissue site is a surface wound, a wound filler may be applied interior to the periwound, and the third layer 505 may be disposed over the periwound and the wound filler. In some embodiments, the filler may be a manifold, such as an open-cell foam. The filler may comprise or consist essentially of the same material as the first layer 205 in some embodiments.

Additionally or alternatively, instillation solution or other fluid may be distributed to the dressing 104, which can increase the pressure in the tissue interface 114. The increased pressure in the tissue interface 114 can create a positive pressure differential across the fluid restrictions 220 in the second layer 210, which can open the fluid restrictions 220 to allow the instillation solution or other fluid to be distributed to the tissue site.

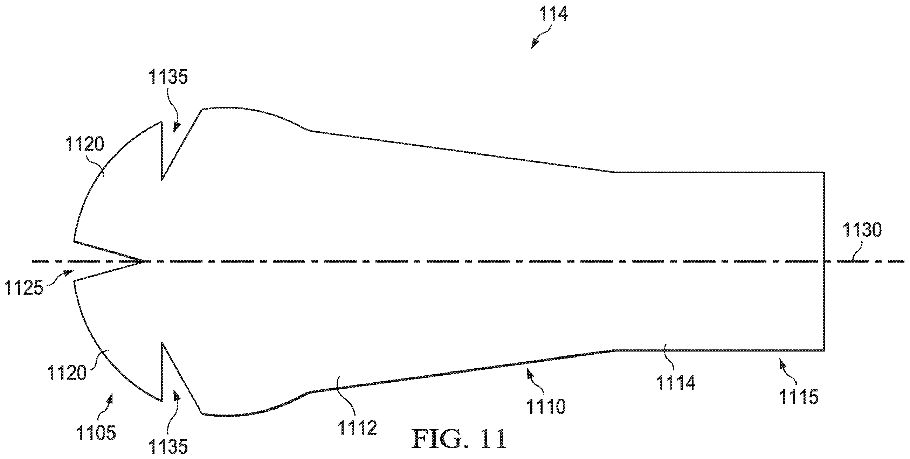

Figure 11 is a plan view of another example of the tissue interface 114, illustrating additional details that may be associated with some embodiments. For example, as illustrated in Figure 11, the tissue interface 114 may have a variety of shapes, which can be adapted for application to specific types of tissue sites. In particular, Figure 11 illustrates an exemplary embodiment of the tissue interface 114 having a profile adapted for use on a foot. Figure 11 illustrates only one layer of the tissue interface 114. Regardless of shape, the tissue interface 114 may comprise more than one layer, as illustrated in previous examples, and the additional layers may have similar profiles and features.

The tissue interface 114 in the example of Figure 11 comprises a hindfoot section 1105; an underfoot section 1110; and a forefoot extension 1115. In some embodiments, the hindfoot section 1105 may be adapted for a heel of the foot. For example, the hindfoot section 1105 may comprise at least two heel flaps 1120. In some embodiments, the underfoot section 1110 may be adapted for the remainder of the foot, for example the midfoot and the forefoot. In some embodiments, the forefoot extension section 1115 may be configured to be folded over at least a portion of the underfoot section 1110. For example, the forefoot extension section 1115 may be configured to be folded over the toes and at least a portion of the bridge of the foot when located in place on the patient's foot. In the example of Figure 11, all of the layers of the tissue interface 114 may span the entirety of the hindfoot section 1105, the underfoot section 1110, and the forefoot extension 1115.

In Figure 11, an anterior part of the hindfoot section 1105 may be attached to a posterior part of the underfoot section 1110, and an anterior part of the underfoot section 1110 may be attached to a posterior part of the forefoot extension section 1115. Thus, the hindfoot section 1105, underfoot section 1110, and forefoot extension section 1115 may be integrally formed, for example as a single, unified, continuous, and/or integrated whole. The tissue interface 114 of Figure 11 may typically be sized and shaped to fit a number of different feet. In some embodiments, the tissue interface 114 may span the entire sole of such feet, while also allowing for portions of the tissue interface 114 to be folded up and/or around the foot.

In some embodiments, the underfoot section 1110 may include a midfoot section 1112 and a forefoot section 1114. In Figure 11, the two heel flaps 1120 of the hindfoot section 1105 may be separated from each other by a hindfoot notch 1125. For example, the hindfoot notch 1125 may be a gap or slit separating the two heel flaps 1120 so as to allow each flap 1120 to be independently folded, disposed, or positioned with respect to the heel of the foot. In some embodiments, the hindfoot notch 1125 may be centered on the longitudinal centerline axis 1130. Also in Figure 11, the hindfoot section 1105 may be separated from the underfoot section 1110 by two demarcation notches 1135. For example, the demarcation notches 1135 may be gaps or slits, which can allow the hindfoot section 1105 to be positioned and/or folded independently of the underfoot section 1110. Typically, the demarcation notches 1135 can be located at mirror-image positions of the tissue interface 114 opposite a longitudinal centerline axis 1130. In some embodiments, the demarcation notches 1135 may each extend inward from the respective exterior side edge of the hindfoot section 1105 towards the longitudinal centerline axis 1130.

In Figure 11, the hindfoot notch 1125 and/or the demarcation notches 1135 may be substantially v-shaped. For example, the hindfoot notch 1125 may be symmetrically shaped, such as a pie-shaped wedge. In some embodiments, the hindfoot notch 1125 may comprise two edges which meet at an acute angle, and the two edges may be substantially identical in length. In some embodiments, each of the demarcation notches 1135 may not be symmetrically shaped. For example, an posterior edge of each demarcation notch 1135 may extend inward substantially perpendicular to the longitudinal centerline axis 1130, while an anterior edge of each demarcation notch 1135 may extend at an acute angle from the posterior edge of the demarcation notch 1135. In some embodiments, the anterior edge of the demarcation notch 1135 may be longer than the posterior edge of the demarcation notch 1135. In some embodiments, the hindfoot section 1105 may have a posterior portion which is curved. So for example, the two heel flaps 1120 of Figure 11 can jointly form approximately a semi-circle, with the diameter of the semi-circle typically approximately matching the maximum width of the tissue interface 114. The hindfoot notch 1125 may be centered on the longitudinal centerline axis 1130 of the tissue interface 114 in some embodiments, extending from the posterior portion of the hindfoot section 1105 inward, longitudinally to approximately the location of the demarcation notches 1135. For example, the posterior edge of the demarcation notches 1135 may be approximately positioned at the same longitudinal position as the anterior portion of the hindfoot notch 1125. And in some embodiments, the anterior edge of the demarcation notches 1135 may project forward toward the posterior portion of the underfoot section 1110.

The forefoot extension section 1115 of Figure 11 may be approximately rectangular in shape. In this context, rectangular may include square shapes. Typically, the width of the underfoot section 1110 decreases as it extends longitudinally from the hindfoot section 1105 to the forefoot extension section 1115. Thus, the underfoot section 1110 is wider at its posterior portion than at its anterior portion in Figure 11. The width may decrease via one or more straight lines, one or more curves, or some combination of curves and lines. For example, in Figure 11 the decreasing width of the underfoot section 1110 may initially occur via curves from the hindfoot section 1105 longitudinally forward, before transitioning to a linear decrease extending longitudinally forward towards the forefoot extension section 1115. For example, in Figure 11 the curves may continue the curvature of the hindfoot section 1105. In some embodiments, the tissue interface 114 may be approximately symmetrical about the longitudinal centerline axis 1130, but an asymmetrical configuration may be appropriate in other embodiments.

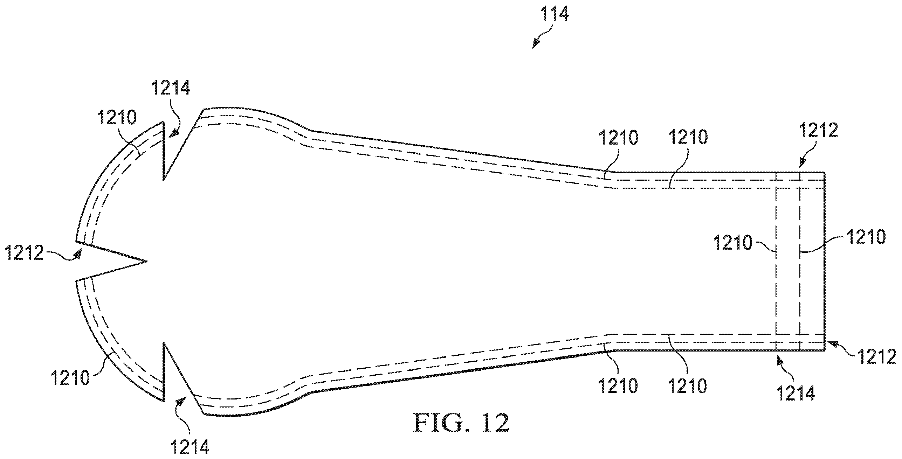

Figure 12 is a plan view of another example of the tissue interface 114, illustrating additional details that may be associated with some embodiments. In Figure 12, the tissue interface 114 includes a plurality of perforations 1210 spaced inward from the perimeter edges of the tissue interface 114 and approximately tracking the perimeter edge contours. So for example, the perforations 1210 may form lines, with the perforations 1210 defining edge portions configured to be removed to size the tissue interface 114 for a specific foot. In some embodiments, the tissue interface 114 may include exterior perforation lines 1212. For example, each exterior perforation line 1212 may be located adjacent to and approximately track the corresponding perimeter edge of the tissue interface 114. In some embodiments, the tissue interface 114 can also have one or more levels of interior perforation lines 1214. For example, each interior perforation lines 1214 may be spaced inward from and approximately track the corresponding exterior perforation line 1212.

In Figure 12, there are a plurality of exterior perforation lines 1212 which jointly track the entire perimeter of the tissue interface 114. Figure 12 also illustrates one exemplary level of interior perforation lines 1214 which jointly track the entire perimeter of the tissue interface 114. In some embodiments, there can be more than one level of such interior perforation lines. In other embodiments, the exterior perforation lines 1212 and/or the interior perforation lines 1214 may span only a portion of the perimeter of the tissue interface 114. For example, the exterior perforation lines 1212 and/or the interior perforation line 1214 may span only the anterior edge of the forefoot extension section 1115 and/or only one side of the underfoot section 1110. In some embodiments, the tissue interface 114 may span the entirety of the hindfoot section 1105, the underfoot section 1110, and the forefoot extension section 1115.

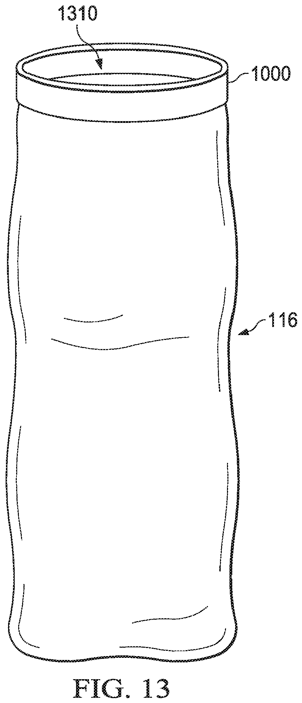

Figure 13 is a perspective view of another example of the cover 116, illustrating additional details that may be associated with some embodiments. In Figure 13, the cover 116 may be preformed and initially separate and apart from the tissue interface 114. In some embodiments, the cover 116 may comprise an open end 1310 configured to receive the tissue interface 114 when applied to an anatomical region, such as a foot. So for example, the remainder of the cover 116, other than the open end 1310, may form a substantially closed surface. Thus, the cover 116 may be bag-like, as shown in Figure 13. In some embodiments, a foot may be placed within and enveloped by the cover 116, for example with the ankle and/or leg extending out the open end 1310. For example, the cover 116 may be a polyurethane bag, which may have a thickness ranging from approximately 100 to 200 micron.

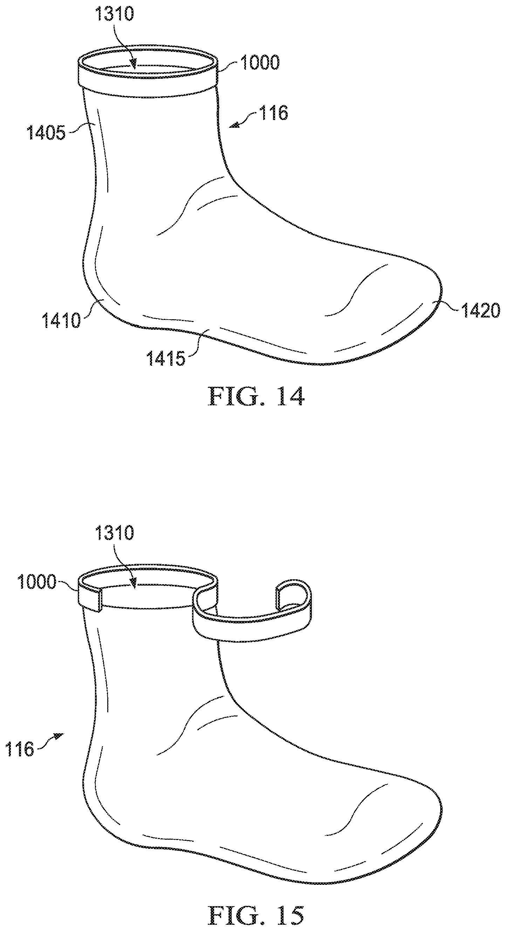

Figure 14 illustrates another example of the cover 116, illustrating additional details that may be associated with some embodiments. In Figure 14, the bag-like cover 116 may be anatomically shaped. For example, the cover 116 of Figure 14 comprises a shape adapted for receiving a foot. So in some embodiments, the cover 116 may have a sock-like shape. In some embodiments, the cover 116 of Figure 14 may include a leg portion 1405, a heel portion 1410, foot portion 1415, and a toe portion 1420. For example, the leg portion 1405 may be configured for the patient's ankle and/or leg, the heel portion 1410 may be configured for the patient's heel, the foot portion 1415 may be configured for a patient's midfoot, and the toe portion 1420 may be configured for the patient's toes and/or forefoot. Typically in use, the patient's foot with applied tissue interface 114 can be inserted into the cover 116 via the opening 1310. The heel portion 1410 of the cover 116 can then interact with the hindfoot portion 1105 of the tissue interface 114. The foot portion 1415 and toe portion 1420 may cover the underfoot section 1110 and/or the forefoot extension section 1115 of the tissue interface 114. The leg portion 1405 of the cover 116 may also cover at least some part of folded-up portions of the hindfoot section 1105 of the tissue interface 114.

In some embodiments, the cover 116 may be constructed by adhering sheets of non-porous material to envelope the tissue interface 114 *in-situ.* For example, one or more sheets may be cut and shaped and then applied over the tissue interface 114 so as to seal the tissue interface 114.

Figure 14 also illustrates another configuration of the attachment device 1000, which can seal the open end 1310 of the cover 116. For example, the attachment device 1000 may attach and seal the cover 116 to the foot, ankle, or leg so as to create a substantially sealed interior environment within the cover 116. In some embodiments, the attachment device 1000 can seal the cover 116 to the patient's skin sufficiently for negative-pressure therapy. For example, the attachment device 1000 may comprise an adhesive cuff around the open end 1310 of the cover 116. Some embodiments may include a release liner (not shown) adapted to be removed from the attachment device 1000 to expose the adhesive of such attachment device 1000.

Figure 15 is a perspective view of an example of the attachment device 1000, illustrating an alternative embodiment in which the attachment device 1000 may be initially separate from the cover 116. In some embodiments, the attachment device 1000 may be configured to be applied to the open end 1310 of the cover 116 and to the foot, ankle, or leg to seal the cover 116 as it encloses the foot. For example, the attachment device 1000 may be an adhesive tape or drape. In some embodiments, the attachment device 1000 may be an approximately 30 mm wide strip.

In some embodiments, the dressing 104 may be provided initially in a pre-packaged kit. Such a kit may be configured to provide a semi-customized dressing, which may allow some customization for a particular type of tissue site, and may significantly reduce the training necessary to properly apply the dressing 104. For example, a kit may comprise the tissue interface 114 and the cover 116. In some embodiments, the tissue interface 114 of the kit can be configured for storage and/or the tissue interface 114 may not be located within the cover 116 during storage. So for example, the tissue interface 114 may be substantially flat and/or unfolded, and the tissue interface 114 and the cover 116 may be located in separate compartments within the kit. Some kit embodiments may also include attachment devices. For example, the attachment device 1000 may be located within the package in a separate compartment from the tissue interface 114 and/or the cover 116. Some kit embodiments may also include a fluid port. While some embodiments may have a fluid port integrated into the cover 116, in other embodiments the fluid port may not be stored within the package pre-attached to the cover 116. So in some embodiments, the fluid port may be configured to be attached after the cover 116 has been taken out of the package and/or has enclosed the tissue interface 114 located on a tissue site.

While some dressing kits may be specifically configured for the foot, similar dressing kits can be configured for other anatomical regions, particularly anatomical regions with complex topography (such as the hand, for example). Such embodiments may typically comprise a tissue interface and a cover, but the tissue interface may be shaped for the specific anatomical region at issue. Thus, each layer may be shaped to co-extensively span various sections of the particular anatomical topography at issue in some embodiments. Furthermore, in some embodiments, the cover may be a bag which is anatomically-shaped for the specific anatomical region at issue. So for example, a cover for a hand dressing kit may be shaped like a glove.

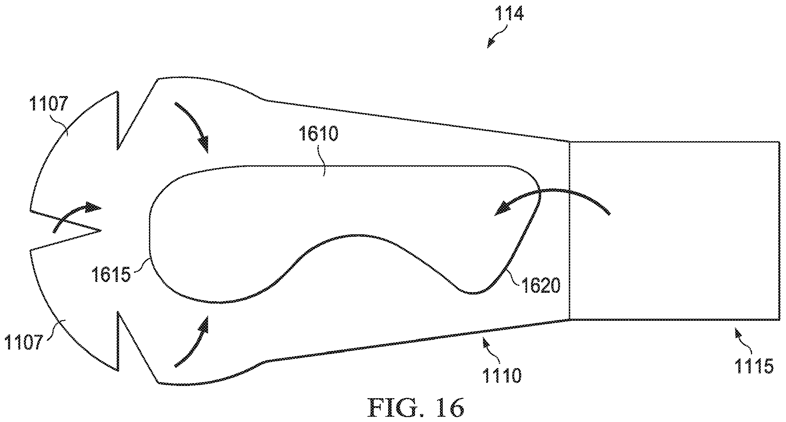

Figure 16 is a plan view of the tissue interface 114 of Figure 11 with an outline of a bottom of a foot 1610 superimposed for illustration purposes. The arrows illustrate one example of how the tissue interface 114 may be folded with respect to the foot 1610. As shown in Figure 16, the sole of the foot 1610 may be placed on the tissue interface 114. For example, the sole of the foot 1610 may contact the underfoot section 1110 of the gel layer 550 or the fluid control layer 210, depending on the configuration of the tissue interface 114. The heel flaps 1120 may be folded up about the posterior edge 1615 of the foot 1610, and the forefoot extension section 1115 may be folded up and over the anterior edge 1620 of the foot 1610. For example, the forefoot extension section 1115 may be folded up and over the forefoot section 1114. In some embodiments, the portions of the underfoot section 1110 which do not underlie the sole of the foot 1610 may be folded up on the foot 1610. So for example, the heel flaps 1120 may be folded up on the heel of the foot 1610, and the forefoot extension section 1115 may be folded over the toes and at least a portion of the bridge of the foot 1610.

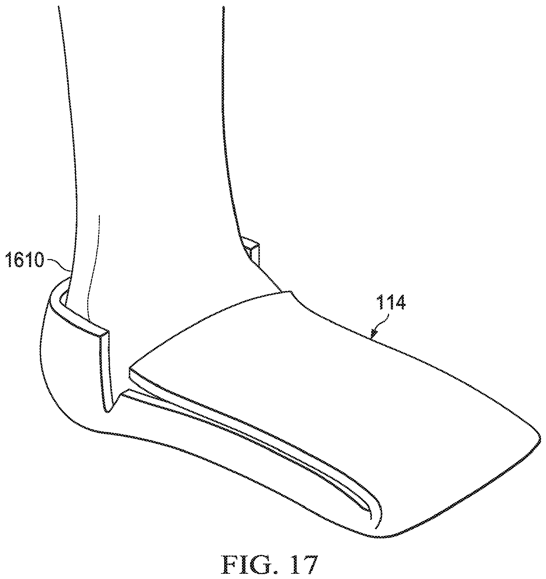

Figure 17 is an isometric view of the tissue interface 114 of Figure 16 when applied to the foot 1610. The tissue interface 114 may substantially encase, enclose, and/or enwrap the foot 1610, as illustrated in the embodiment of Figure 17.

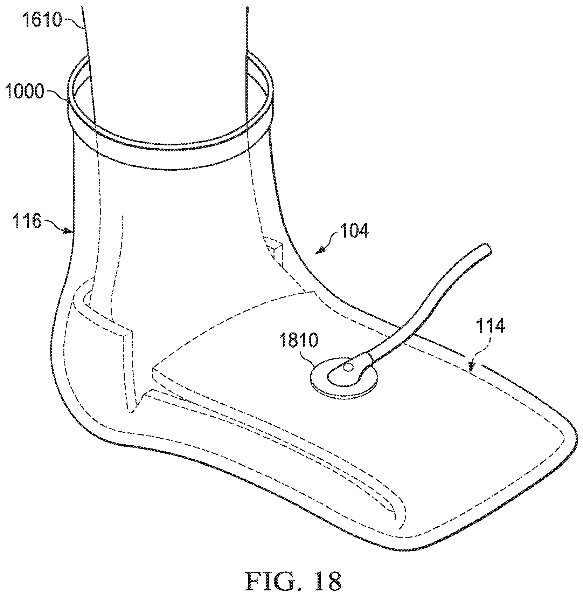

Figure 18 is an isometric view of the tissue interface 114 of Figure 17 with the cover 116 of Figure 14 disposed over the tissue interface 114 and the foot 1610. The attachment device 1000 can seal the cover 115 at the patient's ankle or leg. Once the dressing 104 has been applied to the foot 1610, a fluid port 1810 may be attached to the cover 116. For example, a slit or opening may be formed in the cover 116 at the desired location, and then the fluid port 1810 may be attached over the slit or opening. In other embodiments, the fluid port 1810 may be integrally formed with the cover 116.

In use, the tissue interface 114, cover 116, and/or kit may be configured to simplify application of the dressing 104 to a complex topography of a tissue site. So for example, a method for treating a tissue site on a foot with negative pressure may comprise: (1) providing a tissue interface, where the tissue interface comprises (a) a hindfoot section comprising at least two heel flaps, (b) a midfoot section, (c) a forefoot section, (d) a forefoot extension section configured to fold over the forefoot section; (e) a fluid control layer having a plurality of fluid restrictions, (f) and a manifold adhered to the fluid control layer in a stacked relationship across the hindfoot section, the midfoot section, the forefoot section, and the forefoot extension section; (2) applying the fluid control layer to the sole of the foot so that the heel flaps extend past the posterior edge of the foot and the forefoot extension section extends past the anterior edge of the foot; (3) folding the forefoot extension section over the anterior edge of the foot; (4) folding the heel flaps up at the posterior edge of the foot; (5) placing a cover over the foot and the tissue interface; (6) applying one or more attachment devices to the cover so that the tissue interface is fluidly isolated from the ambient environment; (7) fluidly coupling a negative-pressure source to the tissue interface through the cover; and/or (8) applying negative pressure from a negative-pressure source to the tissue site through the tissue interface. In some embodiments, the midfoot section and the forefoot section may be combined into an underfoot section, and the method may be adapted accordingly. Some embodiments of the method may further comprise providing a cover, wherein the cover is anatomically shaped to approximate the foot.

Some method of use embodiments may comprise: (1) providing a tissue interface 114, the tissue interface comprising: (a) hindfoot section comprising at least two heel flaps, (b) a midfoot section, (c) a forefoot section, (d) a forefoot extension section configured to fold over the forefoot section, (e) a gel layer having a plurality of apertures; (f) a fluid control layer having a plurality of fluid restrictions, and (g) a manifold, wherein the gel layer, the fluid control layer, and the manifold are coupled in a stacked relationship across the hindfoot section, the midfoot section, the forefoot section, and the forefoot extension section, wherein the fluid control layer is disposed between the gel layer and the manifold, and wherein the fluid restrictions are at least partially aligned with the apertures; (2) applying the gel layer to the sole of the foot so that the heel flaps extend past the posterior edge of the foot and the forefoot extension section extends past the anterior edge of the foot; (3) folding the forefoot extension section over the anterior edge of the foot; (4) folding the heel flaps up at the posterior edge of the foot; (5) placing a cover over the foot and the tissue interface; (6) applying one or more attachment devices to the cover so that the tissue interface is fluidly isolated from the ambient environment; (7) fluidly coupling a negative-pressure source to the tissue interface through the cover; and/or (8) applying negative pressure from a negative-pressure source to the tissue site through the tissue interface. In some embodiments, the midfoot section and the forefoot section may be combined into an underfoot section, and the method may be adapted accordingly. Some embodiments of the method may further comprise providing a cover, wherein the cover is anatomically shaped to approximate the foot.

Providing a tissue interface for some method embodiments may further comprise providing a kit comprising the tissue interface and the cover. In some embodiments, providing a kit may include selecting a kit for a particular foot size from a plurality of kits. In some embodiments, the cover may comprise a non-porous film with an open end configured to receive the tissue interface when applied to the foot. Thus, placing a cover over the foot may comprise placing the foot through the open end of the cover. In some embodiments, the cover may be anatomically shaped for a foot, for example with a heel section and a toe section. So in such embodiments, placing a cover over the foot may comprise placing toes of the foot into the toe section and placing a heel of the foot into the heel section. Some method embodiments may further comprise adhering the tissue interface in position on the foot, for example after folding portions of the tissue interface with respect to the foot and/or prior to placing the cover about the tissue interface. For example, the tissue interface may be adhered to the foot by the gel layer adhering to the foot.

In some embodiments, the tissue interface may comprise perimeter perforations defining one or more exterior edge portions of the tissue interface. Thus, some method of use embodiments may further comprise removing one or more exterior edge portions of the tissue interface by tearing or cutting along some or all of the perimeter perforations. For example, such cutting or tearing may serve to size and/or shape the tissue interface for the specific foot at issue.

Methods of manufacturing a dressing may comprise: providing a gel layer having a plurality of apertures; providing a film layer which is fluid impermeable; providing a manifold; attaching the film layer in stacked relationship with the gel layer; attaching the manifold in a stacked relationship with the film layer, opposite the gel layer; and/or perforating the film layer, thereby forming a plurality of fluid restrictions in the film layer. In some embodiments, the plurality of fluid restrictions may at least partially aligned with the plurality of apertures in the gel layer. So for example, each fluid restriction may be in fluid communication with an aperture in the gel layer. In some embodiments, the gel layer may be attached to the film, and the film may be attached to the manifold. In some embodiments, perforating the film layer may result in formation of a fluid control layer. In some embodiments, stacking the gel layer, the fluid control layer, and the manifold may form a tissue interface. In some embodiments, the gel layer, the fluid control layer, and the manifold may be stacked so as to be co-extensive, for example with contacting planar surfaces being sized and shaped identically.

The steps of the manufacturing methods are not restricted as to the order. For example, attaching the film layer in stacked relationship with the gel layer may occur prior to perforating the film layer. In such embodiments, perforating the film layer may occur over the apertures in the gel layer. In some embodiments, perforating the film layer over the apertures in the gel layer may result in perforations that fully penetrate the film and at least partially penetrate the gel layer. In some embodiments, the perforations may fully penetrate the gel layer. This may occur, for example, if the cutting tool for perforating the film to form the fluid restrictions in the fluid control layer is set with a depth to penetrate both the film and the gel layer, for example after the film has been stacked adjacent to the gel layer. In such embodiments, the gel layer may re-seal after perforation.

In some embodiments, perforating the film layer may occur before attaching the manifold in stacked relationship with the film layer. In other embodiments, however, perforating the film layer may occur after attaching the manifold in stacked relationship with the film layer. In some such embodiments, perforating the film layer over the apertures in the gel layer may result in perforations that fully penetrate the fluid control layer and at least partially penetrate the gel layer and the manifold. In some embodiments, the perforations may fully penetrate one or both of the gel layer and the manifold. In such embodiments, the gel layer may re-seal, and the manifold may still function effectively. Some method of manufacturing embodiments may further comprise perforating the gel layer to form the apertures. This may occur before attaching the film layer in stacked relationship with the gel layer.

Method of manufacturing embodiments may further comprise shaping the tissue interface anatomically for interaction with a foot, such that the tissue interface may comprise: a hindfoot section comprising at least two heel flaps; an underfoot section adapted to a remainder of the foot; and a forefoot extension section configured to fold over at least a portion of the underfoot section. In some embodiments, shaping may comprise cutting, punching, stamping, molding, etc. the tissue interface. In some embodiments, the gel layer, fluid control layer, and the manifold may span the entirety of the hindfoot section, the underfoot section, and the forefoot extension section. In some embodiments, shaping the tissue interface may comprise shaping each of the gel layer, the fluid control layer, and manifold. In other embodiments, the layers may be pre-attached in stacked relationship before shaping occurs, so that the entire tissue interface may be shaped simultaneously.

In some embodiments, shaping the tissue interface 114 may comprise forming two heel flaps 1120 in the hindfoot section 1105. For example, forming two heel flaps 1120 may comprise cutting two demarcation notches 1135 for separating each flap 1120 from the underfoot section 1110, and a hindfoot notch 1125 for separating the two flaps 1120 from each other. Some embodiment may further comprise providing a release liner 245, and releasably attaching the release liner 245 to the gel layer 505. In some embodiments, the release liner 245 may be attached after perforating the film layer to form the fluid control layer 210.

Some method of manufacture embodiments may further comprise providing a cover. Some method embodiments may further comprise applying the attachment device to the cover. Providing a cover may include providing or forming a cover which is bag-like with an open end, in some embodiments. And in some embodiments, forming a cover may further comprise forming the bag-like cover to have an anatomical shape, such as a foot or sock shape. Some embodiments may further comprise packing the cover and the tissue interface within a package to form a kit. Some embodiments may also include forming a plurality of kits of different sizes. Such embodiments may further comprise selecting a tissue interface and a cover of matching, corresponding, and/or appropriate size.

The systems, apparatuses, and methods described herein may provide significant advantages. For example, anatomically-shaped embodiments of the tissue interface 114 may simplify application of the tissue interface 114 to a tissue site having a complex topography, such as a foot. For example, given the complexities of the topography of the foot region, and the fact that there can be such variance from patient-to-patient and/or that a wound may be located anywhere across the foot or even at more than one place across the foot surface simultaneously, anatomical-configured embodiments of the dressing 104 that can better and more easily adapt to the foot may be beneficial.

The dressing 104 may also reduce the need for extensive customization. For example, some embodiments of the dressing 104 may minimize the need for cutting and shaping of the tissue interface 114 and/or the cover 116, to achieve a proper fit for negative pressure therapy. The level of training necessary for a caregiver to effectively apply the dressing 104 may also be reduced, and/or the need for multiple caregivers to apply the dressing 104 may be reduced. For example, some embodiments of the tissue interface 114 may have an adhesive surface configured for contact with the patient's foot, and this adhesive quality may eliminate the need for an extra pair of hands during application of the tissue interface 114. For example, some embodiments of the tissue interface 114 may have a gel layer configured to hold the tissue interface 114 in place after it is folded up and/or about the foot, prior to application of the cover 116.

In some embodiments, the dressing 104 may be configured to remain in place for extended durations, while minimizing maceration around a tissue site. This ability to safely stay in place on a tissue site may be helpful for healing, minimizing trauma associated with removal of the dressing and reducing the need for complex dressing replacement. In some embodiments, a bag-like cover can simplify application of the cover to the patient's foot, further simplifying application of the dressing 104. This advantage may be further enhanced by an anatomically-shaped bag-like cover, which may simplify application of the cover still further and/or enable improved cover fit about the patient's foot. In some embodiments, the cover may be configured to allow the foot to breathe, even while providing an effective sealed environment for negative pressure therapy, which may help to reduce foot odor and skin maceration.

## Claims

1. A dressing for use with negative-pressure treatment on a foot, the dressing comprising:
a tissue interface (114) comprising:
a hindfoot section (1105) comprising at least two heel flaps (1120),
a midfoot (1112) section,
a forefoot (1114) section,
a forefoot extension (1115) configured to fold over the forefoot section,
a fluid control layer having a plurality of fluid restrictions, and
a manifold adhered to the fluid control layer in a stacked relationship across the hindfoot section (1105), the midfoot section (1112), the forefoot section (1114), and the forefoot extension (1115); and
a cover (116) comprising a non-porous film having an open end (1310) configured to receive the tissue interface applied to the foot.

2. The dressing of claim 1, further comprising a gel layer disposed adjacent to the fluid control layer opposite the manifold, the gel layer having a plurality of apertures at least partially aligned with the fluid restrictions of the fluid control layer.

3. The dressing of claim 2, wherein the gel layer comprises a hydrophobic gel and has a hardness of between about 5 Shore OO and about 80 Shore OO.

4. The dressing claim 2, wherein the gel layer comprises silicone gel, preferably with an area density less than 300 grams per square meter.

5. The dressing of claim 1, further comprising a fluid port configured to be fluidly coupled to the manifold through the cover.

6. The dressing of claim 1, further comprising an attachment device configured to seal the open end of the cover to a lower portion of a leg above the foot.

7. The dressing of claim 1, wherein the fluid control layer comprises a polymer film, a hydrophobic film, a polyethylene film, or a polyurethane film.

8. The dressing of claim 1, wherein the fluid restrictions comprise a plurality of slots, each of the slots having a length less than 4 millimeters.

9. The dressing of claim 1, wherein the fluid restrictions comprise a plurality of slots, each of the slots having a length less than 4 millimeters and a width less than 2 millimeters.

10. The dressing of claim 1, wherein the fluid restrictions comprise or consist essentially of elastomeric valves and the elastomeric valves are normally closed.

11. The dressing of claim 10, wherein the elastomeric valves are fenestrations or slits.

12. The dressing of claim 14, wherein the fluid restrictions comprise a plurality of slits, each of the slits having a length less than 4 millimeters.

13. The dressing of claim 1, wherein the manifold comprises foam, a polymer foam, a polyurethane ether foam, an open-cell foam, a reticulated foam, a reticulated polymer foam, a reticulated polyurethane ether foam, a reticulated foam having a free volume of at least 90%, porous foam having an average pore size in a range of 400-600 microns.

14. The dressing of claim 1, wherein the manifold has a thickness less than 7 millimeters, and preferably in the range of 2 millimeters to 7 millimeters.

15. The dressing of claim 1, wherein the manifold is hydrophobic.

## Patentansprüche

1. Ein Verband zur Verwendung mit einer Unterdruckbehandlung auf einem Fuß, der Verband aufweisend:
eine Gewebeschnittstelle (114), aufweisend:
einen Rückfußabschnitt (1105), aufweisend mindestens zwei Fersenlaschen (1120),
einen Mittelfuß(1112)-Abschnitt,
einen Vorderfuß(1114)-Abschnitt,
eine Vorderfußverlängerung (1115), die konfiguriert ist, um sich über den Vorderfußabschnitt zu falten,
eine Fluidkontrollschicht, die eine Vielzahl von Fluidbeschränkungen aufweist, und
einen Verteiler, der an der Fluidkontrollschicht in einer gestapelten Beziehung über den Rückfußabschnitt (1105), den Mittelfußabschnitt (1112), den Vorderfußabschnitt (1114) und die Vorderfußverlängerung (1115) festgemacht ist; und
eine Abdeckung (116), aufweisend einen nicht porösen Film, die ein offenes Ende (1310) aufweist, das konfiguriert ist, um die Gewebeschnittstelle aufzunehmen, die auf den Fuß aufgebracht ist.

2. Der Verband nach Anspruch 1, ferner aufweisend eine Gelschicht, die angrenzend an die Fluidkontrollschicht gegenüber dem Verteiler angeordnet ist, wobei die Gelschicht eine Vielzahl von Öffnungen aufweist, die mindestens teilweise an den Fluidbeschränkungen der Fluidkontrollschicht ausgerichtet sind.

3. Der Verband nach Anspruch 2, wobei die Gelschicht ein hydrophobes Gel aufweist und eine Härte zwischen etwa 5 Shore OO und etwa 80 Shore OO aufweist.

4. Der Verband nach Anspruch 2, wobei die Gelschicht Silikongel aufweist, vorzugsweise mit einer Flächendichte von weniger als 300 Gramm pro Quadratmeter.

5. Der Verband nach Anspruch 1, ferner aufweisend einen Fluidanschluss, der konfiguriert ist, um mit dem Verteiler durch die Abdeckung fluidisch gekoppelt zu werden.

6. Der Verband nach Anspruch 1, ferner aufweisend eine Befestigungsvorrichtung, die konfiguriert ist, um das offene Ende der Abdeckung an einem unteren Abschnitt eines Beins über dem Fuß abzudichten.

7. Der Verband nach Anspruch 1, wobei die Fluidkontrollschicht eine Polymerfolie, eine hydrophobe Folie, eine Polyethylenfolie oder eine Polyurethanfolie aufweist.

8. Der Verband nach Anspruch 1, wobei die Fluidbeschränkungen eine Vielzahl von Schlitzen aufweisen, wobei jeder Schlitz eine Länge von weniger als 4 Millimetern aufweist.

9. Der Verband nach Anspruch 1, wobei die Fluidbeschränkungen eine Vielzahl von Schlitzen aufweisen, wobei jeder Schlitz eine Länge von weniger als 4 Millimetern und eine Breite von weniger als 2 Millimetern aufweist.

10. Der Verband nach einem Anspruch 1, wobei die Fluidbeschränkungen elastomere Ventile aufweisen oder im Wesentlichen aus solchen bestehen, und wobei die elastomeren Ventile üblicherweise geschlossen sind.

11. Der Verband nach Anspruch 10, wobei die elastomeren Ventile Fenestierungen oder Schlitze sind.

12. Der Verband nach Anspruch 14, wobei die Fluidbeschränkungen eine Vielzahl von Schlitzen aufweisen, wobei jeder Schlitz eine Länge von weniger als 4 Millimetern aufweist.

13. Der Verband nach Anspruch 1, wobei der Verteiler Schaumstoff, einen Polymerschaum, einen Polyurethanetherschaum, einen offenzelligen Schaum, einen retikulierten Schaum, einen retikulierten Polymerschaum, einen retikulierten Polyurethanetherschaum, einen retikulierten Schaum, der ein freies Volumen von mindestens 90 % aufweist, porösen Schaum, der eine durchschnittliche Porengröße in einem Bereich von 400-600 Mikrometer aufweist, aufweist.

14. Der Verband nach Anspruch 1, wobei der Verteiler eine Dicke von weniger als 7 Millimetern und vorzugsweise im Bereich von 2 Millimeter bis 7 Millimeter aufweist.

15. Der Verband nach Anspruch 1, wobei der Verteiler hydrophob ist.

## Revendications

1. Pansement destiné à être utilisé avec un traitement par pression négative sur un pied, le pansement comprenant :
une interface avec du tissu (114) comprenant :
une section d'arrière-pied (1105) comprenant au moins deux rabats de talon (1120),
une section de milieu de pied (1112),
une section d'avant-pied (1114),
une extension d'avant-pied (1115) configurée pour se plier sur la section d'avant-pied,
une couche de régulation de fluide ayant une pluralité de restrictions de fluide, et
un collecteur collé à la couche de régulation de fluide en une relation empilée sur toute la section d'arrière-pied (1105), la section de milieu de pied (1112), la section d'avant-pied (1114), et l'extension d'avant-pied (1115) ; et
un recouvrement (116) comprenant un film non poreux ayant une extrémité ouverte (1310) configurée pour recevoir l'interface avec du tissu appliquée sur le pied.

2. Pansement selon la revendication 1, comprenant en outre une couche de gel disposée adjacente à la couche de régulation de fluide à l'opposé du collecteur, la couche de gel ayant une pluralité d'ouvertures au moins partiellement alignées avec les restrictions de fluide de la couche de régulation de fluide.

3. Pansement selon la revendication 2, dans lequel la couche de gel comprend un gel hydrophobe et a une dureté comprise entre environ 5 Shore OO et environ 80 Shore OO.

4. Pansement selon la revendication 2, dans lequel la couche de gel comprend du gel de silicone, de préférence avec une masse surfacique inférieure à 300 grammes par mètre carré.

5. Pansement selon la revendication 1, comprenant en outre un orifice de fluide configuré pour être couplé de manière fluidique au collecteur à travers le recouvrement.

6. Pansement selon la revendication 1, comprenant en outre un dispositif d'attache configuré pour sceller l'extrémité ouverte du recouvrement sur une partie inférieure d'une jambe au-dessus du pied.

7. Pansement selon la revendication 1, dans lequel la couche de régulation de fluide comprend un film de polymère, un film hydrophobe, un film de polyéthylène, ou un film de polyuréthane.

8. Pansement selon la revendication 1, dans lequel les restrictions de fluide comprennent une pluralité de fentes, chacune des fentes ayant une longueur inférieure à 4 millimètres.

9. Pansement selon la revendication 1, dans lequel les restrictions de fluide comprennent une pluralité de fentes, chacune des fentes ayant une longueur inférieure à 4 millimètres et une largeur inférieure à 2 millimètres.

10. Pansement selon la revendication 1, dans lequel les restrictions de fluide comprennent ou sont constituées sensiblement de valves élastomères et les valves élastomères sont normalement fermées.

11. Pansement selon la revendication 10, dans lequel les valves élastomères sont des fenêtres ou des entailles.

12. Pansement selon la revendication 14, dans lequel les restrictions de fluide comprennent une pluralité d'entailles, chacune des entailles ayant une longueur inférieure à 4 millimètres.

13. Pansement selon la revendication 1, dans lequel le collecteur comprend de la mousse, une mousse de polymère, une mousse de polyuréthane-éther, une mousse à alvéoles ouvertes, une mousse réticulée, une mousse de polymère réticulé, une mousse de polyuréthane-éther réticulé, une mousse réticulée ayant un volume libre d'au moins 90 %, une mousse poreuse ayant une taille moyenne des pores dans une plage de 400 à 600 micromètres.

14. Pansement selon la revendication 1, dans lequel le collecteur a une épaisseur inférieure à 7 millimètres, et de préférence dans la plage de 2 millimètres à 7 millimètres.

15. Pansement selon la revendication 1, dans lequel le collecteur est hydrophobe.
